# EUROPÄISCHE PATENTSCHRIFT

(11) **EP 3 046 457 B1**
(45) Veröffentlichungstag und Bekanntmachung des Hinweises auf die Patenterteilung: **24.04.2019**
(21) Anmeldenummer: 14801927.6
(22) Anmeldetag: 18.09.2014
(51) Int. Cl.: A61B 1/247, A61C 17/06, A61B 1/253

(54) **SPIEGELSAUGER MIT FESTEM SPIEGEL**
MIRROR SUCKER HAVING A SOLID MIRROR
POMPE À SALIVE À MIROIR POURVUE D'UN MIROIR FIXE

(30) Priorität: 18.09.2013 DE 102013110302
(43) Veröffentlichungstag der Anmeldung: 27.07.2016
(73) Patentinhaber: Cleverdent Ltd., 48149 Münster (DE)
(72) Erfinder: Clasen, Stephan, 48149 Münster (DE); Kayser, Martin, 50968 Köln (DE)
(74) Vertreter: Patentanwälte Bauer Vorberg Kayser
(86) Internationale Anmeldenummer: PCT/EP2014/002529
(87) Internationale Veröffentlichungsnummer: WO 2015/039752

(56) Entgegenhaltungen:
- EP-A1- 0 314 657
- DE-B3-102012 100 119
- US-A- 3 928 916

## Beschreibung

Die vorliegende Erfindung betrifft einen zahnmedizinischen Spiegelsauger zum Absaugen von Flüssigkeiten und Partikeln aus einem Mundraum eines Patienten, mit einem hohlen Grundkörper, der eine Außenfläche, eine Innenfläche, eine Längsachse, eine Anschlussöffnung und eine Ansaugöffnung aufweist, wobei die Innenfläche einen durch die Ansaugöffnung einsehbaren Spiegel aufweist und der Grundkörper aus einem ersten Grundkörperteil und einem zweiten Grundkörperteil gebildet ist, die fest miteinander verbunden sind. Weiterhin betrifft die Erfindung ein Verfahren zur Herstellung eines solchen Spiegelsaugers.

Bei zahnmedizinischen Behandlungen ist es oftmals notwendig, anfallende Flüssigkeiten oder gelöste Partikel, beispielsweise Speichel, Spraywasser und Blut während der Behandlung abzusaugen. Auch kann Wasser, beispielsweise zum Reinigen oder nach Anwenden einer Multifunktionsspritze anfallen, das abgesaugt werden muss. Hierzu werden üblicherweise Absauger verwendet, die in der Regel aus einem röhrenförmigen Körper aus Kunststoff gebildet sind, an dessen Ende ein Schlauch befestigt ist, der wiederum mit einer Pumpe verbunden ist. Durch den Schlauch werden die störenden Flüssigkeiten und Festkörper abgeleitet.

Oftmals wird ein Absauger nicht vom behandelnden Zahnarzt oder Zahnchirurgen selbst, sondern von einer oder einem Assistenten geführt und gehalten, weil der behandelnde Zahnarzt mit der einen Hand ein Bohrwerkzeug und mit der anderen Hand einen Spiegel, über den er den zu behandelnden Bereich einsehen kann, halten muss. Nachteilig bei der beschrieben Vorgehensweise ist, dass die beiden Personen sehr eng beieinander um den zu behandelnden Bereich stehen oder sitzen müssen. Dies kann gerade dann, wenn es sich um verhältnismäßig schwierige oder feinmotorisch anspruchsvolle Eingriffe handelt, für den behandelnden Arzt als störend empfunden werden.

Aus der DE 102006048463 A1 ist ein medizinischer Spiegelsauger bekannt, bei dem die Innenfläche eine durch die Ansaugöffnung sichtbare verspiegelte Oberfläche aufweist. Die erfindungsgemäße spiegelnde Beschichtung ermöglicht es dem Benutzer, den medizinischen Spiegelsauger sowohl als Spiegelsauger zum Abführen von Flüssigkeiten und Partikeln, als auch gleichzeitig als Spiegel zu benutzen. Mit Hilfe eines solchen Spiegelsaugers ist es ihm nun möglich, die Behandlung ohne eine assistierende Person durchzuführen. Der Spiegelsauger wird also gleichzeitig als Absauger und als Spiegel verwendet.

Die Grundidee der Kombination der beiden Geräte ist grundsätzlich sehr gut, allerdings hat sich die praktische Umsetzung der Idee bzw. die Herstellung eines solchen Spiegelsaugers als äußerst schwierig herausgestellt. Der Spiegel muss fest eingefasst sein, er darf sich nicht lösen und sollte sich auch während der Behandlung nicht bewegen dürfen, da dadurch die Sicht beeinträchtigt wird. Auch dürfen möglichst keine Unebenheiten oder Spalte vorhanden sein, da diese die Geräuschentwicklung negativ beeinflussen.

Druckschrift EP 0 314 657 A1 zeigt beispielsweise eine Kombination aus Absauger und Spiegel, bei dem der Spiegel in ein mehrteiliges Spiegelhalterelement eingesetzt ist. Die Ansaugluft wird durch relativ enge Spalte um den Spiegel herum und durch eine Öffnung hinter dem Spiegel in einen Hohlkörper geleitet. Aufgrund der geringen Spalte eignet sich der Dentalspiegel nicht für das Absaugen größerer Partikel. Der Aufbau ist relativ kompliziert und die Ansaugleistung gering.

In Dokument US 3 928 916 ist ein Handspiegel gezeigt, der auf seiner Rückseite, also hinter dem Spiegel, Ansaugöffnungen für Luft aufweist. Somit müssen Partikel zunächst im Mundraum am Handspiel vorbei nach unten fallen, bevor sie angesaugt werden können. Hinzu kommt, dass auch bei dieser Ausführung die Ansaugöffnungen sehr klein sind.

Der Grundkörper des Spiegelsaugers kann, wie beispielsweise in DE 10 2012 100 119 B3 gezeigt, aus zwei verschweißten Längshälften gebildet sein, die den Spiegel in einer Nut halten. Ein spaltfreies Verschweißen der Längshälften ist aber kaum zufriedenstellend möglich, es wird entweder eine Schattenfuge gebildet oder es entsteht beim Schweißvorgang Materialüberschuss, der anschließend abgetragen werden muss. Die Schattenfuge ist unschön und es können sich Flüssigkeiten und Bakterien in ihr sammeln, der Abtrag des Materialüberschusses ist zeitaufwändig und führt zu sichtbaren Veränderungen der Oberfläche des Grundkörpers. Die beiden Längshälften müssen darüber hinaus ausgesprochen exakt gefertigt sein, um nach dem Zusammensetzen bzw. Verschweißen eine ansprechende, insbesondere spalt- und/oder überschussfreie Erscheinung zu gewährleisten. Diese sind vor allem deswegen störend, weil der behandelnde Zahnarzt im Längsverlauf des Spiegelsaugers jede Unebenheit, Nut oder Grad mit den Figern spürt. Insbesondere sind auch in Längsrichtung nur geringste Längentoleranzen der beiden Hälften akzeptabel, die beiden Hälften dürfen darüber hinaus in Längsrichtung auch nicht minimal versetzt zueinander angeordnet sein.

Die Aufgabe der vorliegenden Erfindung besteht darin, einen Spiegelsauger bereitzustellen, der eine ausreichend hochwertige Spiegelfläche im Bereich der Ansaugöffnung aufweist. Der Spiegelsauger soll dabei kostengünstig herstellbar sein und ein ansprechendes Äußeres aufweisen. Die Nachteile des Standes der Technik sollen vermieden werden, insbesondere soll der Spiegelsauger keine oder nur unwesentliche Spalte auf seiner Außenseite aufweisen. Weiterhin ist es

Aufgabe der Erfindung, ein Verfahren zur Herstellung eines Spiegelsaugers mit den genannten Vorteilen vorzuschlagen.

Erfindungsgemäß wird die Aufgabe durch einen Spiegelsauger mit den Merkmalen des Patentanspruchs 1 gelöst. Ein erfindungsgemäßes Herstellungsverfahren ist in Patentanspruch 10 angegeben.

Demnach ist der Spiegel aus zwei Grundkörperteilen gebildet, die miteinander verbunden, vorzugsweise verschweißt oder verklebt sind und so einen einstückigen Spiegelsauger ausbilden. Einstückig im Sinne der Erfindung bedeutet, dass die beiden Grundkörperteile nach der Fertigung nur durch Zerstören wieder voneinander getrennt werden können.

Der Spiegel ist fest im Spiegelsauger befestigt, was insofern vorteilhaft ist, weil der Grundkörper und der Spiegel im Praxisbetrieb als Einheit behandelt werden können. Der Spiegel und Spiegelsauger können gemeinsam sterilisiert werden, ein Einsetzen und Auslösen des Spiegels im Praxisbetrieb entfällt.

Der Begriff "fest" bedeutet in diesem Zusammenhang, dass der Spiegel unverlierbar gehalten ist, er kann nicht herausgelöst werden, ohne den Spiegelsauger oder den Spiegel zu zerstören.

Eine wesentliche Erkenntnis besteht darin, dass ein ansprechendes Äußeres des Grundkörpers dann erreicht wird, wenn eines der beiden ersten Grundkörperteile möglichst groß und das andere Grundkörperteil möglichst klein ist und sich das kleinere Grundkörperteil nur in geringem Maße in Längsrichtung des Spiegelsaugers erstreckt. Dadurch ist die durch die Verbindungsflächen zwischen den Grundkörperteilen entstehende störende Nut oder der störenden Grad relativ kurz.

Weiterhin ist entscheidend, dass das kleinere Grundkörperteil und die Verbindung zwischen den Grundkörperteilen in einem Bereich angeordnet sind, den der behandelnde Arzt während der Behandlung nicht oder nur wenig berührt. Selbst wenn die Verbindungsflächen die Oberfläche des Spiegelsagers negativ beeinflussen, führen sie nicht zu einer haptischen Störung.

Die Anordnung im Bereich der Rückseite des Spiegels, also an der Unterseites des Spiegelsaugers ist deshalb besonders vorteilhaft, weil dieser Bereich in der Regel beim Gebrauch des Spiegelsaugers nicht einsehbar ist. Auch nicht ertastbare, aber sichtbare Veränderungen der Oberfläche fallen somit kaum negativ auf.

Das erste Grundkörperteil bildet somit nahezu den gesamten Grundkörper des Spiegelsaugers aus, während das zweite Grundkörperteil im Wesentlichen lediglich die zum Einsetzen des Spiegels in das erste Grundkörperteil notwendige Öffnung verschließt. Bezogen auf die äußere Oberfläche hat das erste Grundkörperteil einen Flächenanteil von 80 bis 95 %, das zweite Grundkörperteil einen Flächenanteil von 5 bis 20 %.

Der Spiegel ist vorteilhafterweise rund, kann aber auch oval sein oder andere geeignete Formen aufweisen. Im Folgenden wird von der üblichen runden Form des Spiegels ausgegangen.

Um den Spiegel auf Dauer zuverlässig zu halten, weist das erste Grundkörperteil eine Spiegelaufnahme mit einer Öffnung zum Einsetzen des Spiegels auf. Das erste Grundkörperteil umgibt also seitlich den in die Öffnung eingesetzten Spiegel. Die Öffnung ist beim fertigen Spiegelsauger auf der Rückseite, also hinter dem eingesetzten Spiegel durch das zweite Grundkörperteil verschlossen. Die beiden Grundkörperteile sind miteinander verschweißt oder verklebt.

Das den Spiegel umgebende und ihn haltende Material ist vorteilhafterweise weich oder elastisch, so dass es Materialausdehnungen, die beispielsweise bei der Sterilisation entstehen ausgleichen kann. Ungewollte Spannungen im Bereich des Spiegels, die den Spiegel und/oder den Grundkörper zerstören können, werden dadurch wirkungsvoll vermieden. Alternativ oder zusätzlich kann der Spiegel in einer Nut gehalten sein, die in seitlicher Richtung ausreichend tief ist, um eine Wärmeausdehnung bzw. eine Vergrößerung der Fläche und des Durchmessers des Spiegels auszugleichen. Der sich ausdehnende Spiegel kann sich in die ausrechend tiefe Nut hinein erstrecken. Anstelle der Nut kann auch der Durchmesser der Öffnung im ersten Grundkörperteil, in die der Spiegel eingesetzt ist, einen etwas größeren Durchmesser als der Spiegel aufweisen.

Die Sichtfläche des Spiegels bleibt frei und ist von vorne einsehbar. Das auf der Rückseite des Spiegels angeordnete zweite Grundkörperteil weist in einer vorteilhaften Ausführungsvariante insgesamt Abmessungen auf, die nur unwesentlich über die Abmessungen des Spiegels hinausgehen. Dadurch ist es möglich, zunächst das erste Grundkörperteil zu fertigen, den Spiegel dann von hinten in die freie Öffnung in das erste Grundkörperteil einzusetzen und schließlich die Öffnung mit dem zweiten Grundkörperteil von hinten zu verschließen.

Erfindungsgemäß verjüngt sich die Öffnung im ersten Grundkörperteil in Richtung einer Bodenfläche innerhalb des Grundkörpers im Bereich der Ansaugöffnung, sie ist derart ausgeführt und bemessen, dass der eingesetzte Spiegel in der Spiegelaufnahme an einer oberen Halteschulter anliegt, die in einer Innenwandung der Öffnung ausgebildet ist.

Wesentlich ist, dass die Öffnung der Spiegelaufnahme des ersten Grundkörperteils derart dimensioniert ist, dass der Spiegel in die Spiegelaufnahme einführbar ist. Die Öffnung weist deshalb auf ihrer der Grundkörperrückseite zugewandten Seite einen Durchmesser auf, der den Durchmesser des Spiegels übersteigt. Dementsprechend weist auch das zweite Grundkörperteil einen Durchmesser auf, der den Durchmesser des Spiegels übersteigt. Letztendlich ist die Öffnung in vertikaler Querschnittsrichtung ein konisch zulaufender Kanal, in dem der Spiegel von der breiteren Seite her eingesetzt wird. Diese Ausführungen beziehen sich auf eine runde Grundform des Spiegels, weist dieser eine andere Form auf, muss die Spiegelaufnahme derart ausgeführt sein, dass auch diese andere Form aufnehmbar ist.

In einer besonders vorteilhaften Ausführungsvariante ist der Spiegel in vertikalen Querschnitt im Wesentlichen trapezförmig ausgeführt, wobei der Durchmesser des Spiegels ausgehend von einer Spiegelfläche in Richtung der Grundkörperunterseite zunimmt. Wie bereits ausgeführt weist die Innenwandung der Öffnung des ersten Grundkörperteils einen dazu korrespondierenden vertikalen Querschnitt auf, ihr Durchmesser nimmt ausgehend von der Grundkörperunterseite zu.

Die trapezförmige Form der äußeren Wandung des Spiegels und der Innenwandung der Öffnung bzw. die obere Halteschulter sind vorteilhafterweise derart gewählt, dass die sichtbare Spiegeloberfläche im eingesetzten Zustand des Spiegels bündig mit der Bodenfläche des ersten Grundkörperteils, die die Spiegelfläche umgibt, abschließt. Die obere Halteschulter verhindert durch die Anlage an der Außenseite des Spiegels, dass der Spiegel nach oben über die Bodenfläche überstehen oder sich in diese Richtung aus dem Grundkörper lösen kann.

Vorteilhafterweise ist der Spiegel bereits beim Einsetzen in der Spiegelaufnahme reib- oder formschlüssig gehalten. Beispielsweise kann der Durchmesser der Öffnung minimal geringer ausgeführt sein, als der Durchmesser des Spiegels. Der Spiegel verformt dann beim Einsetzen das umgebende Material, drückt es etwas zurück, so dass der Spiegel anschließend durch das elastische Material gehalten wird. Anschließend wird das zweite Grundkörperteil an das erste Grundkörperteil mit dem eingesetzten und gehaltenen Spiegel angeklebt oder angespritzt.

Alternativ ist auch denkbar, dass nicht der gesamte Durchmesser der Öffnung geringer als der Durchmesser des Spiegels ist, sondern das lediglich mehrere, vorzugsweise drei über den Verlauf der Innenwandung oder Halteschulter gleichmäßig verteilte Erhöhungen vorgesehen sind, die den Spiegel bereits vor der Verbindung mit dem zweiten Grundkörperteil in seiner Position halten.

Die Spiegeloberfläche und die umgebende Bodenfläche bilden eine möglichst plane Gesamtfläche, über die der Luftstrom, angesaugte Flüssigkeit und Partikel optimal abgeführt werden können. Die plane Gesamtfläche bewirkt auch, dass die Geräuschentwicklung durch Luftverwirbelungen in diesem Bereich gering ist. Ein Überstand des Spiegels gegenüber der Bodenfläche des ersten Grundkörperteils von bis zu 0,3 mm wird im Sinne der Erfindung noch als bündig angesehen.

Alternativ zur reinen Trapezform kann der Spiegel etwa im Mittelbereich seines vertikalen Querschnitts einen maximalen Durchmesser aufweisen. Der Durchmesser nimmt dann also von der Spiegelfläche ausgehend zunächst zu und anschließend in Richtung der Rückseite des Spiegels wieder ab. Die Innenwandung der Öffnung ist dann korrespondierend ausgebildet, so dass der Spiegel in die sich ausgebildete Spiegelhalterungsnut eingeklipst werden kann. Die Innenwanddung der Öffnung bildet dann nicht nur eine obere Halteschulter, sondern auch eine untere Halteschulter aus. Denkbar ist auch, dass die untere Halteschulter, die den Spiegel ausgehend von seiner Rückseite noch vor seinem maximalen Durchmesser kontaktiert, durch das zweite Grundkörperteil gebildet ist.

Das zweite Grundkörperteil kann derart mit dem ersten Grundkörperteil verbunden werden und eine entsprechende Form aufweisen, dass dieses den Spiegel innerhalb der Öffnung unter Vorspannung gegen die obere Halteschulter drückt. Dies gewährleistet, dass der Spiegel sicher gehalten ist und sich auch während der Behandlung nicht bewegen kann. Die Innenwandung der Öffnung wirkt als Dichtlippe und legt sich ähnlich wie ein Simmering umlaufend an die Spiegelseitenfläche an.

Für die Fertigung eignet sich insbesondere thermoplastischer Kunststoff zum Beispiel Polypropylen oder auch Polyethylen. Durch die Hinzugabe von Zusatzstoffen kann die äußere Erscheinung des Spiegelsaugers beeinflusst werden. Erfindungsgemäß hat sich auch die Fertigung aus Polyester als vorteilhaft erwiesen. Polyester weist hinsichtlich der Oberflächenbeschaffenheit des fertigen Produkts gegenüber anderen Kunststoffen deutliche Vorteile beispielsweise hinsichtlich des einstellbaren Glanzgrades, der Kratzfestigkeit sowie der Oberflächenglätte auf. Als geeigneter Zuschlagstoff zur Beeinflussung der Materialeigenschaften kommen beispielsweise Glaskugeln in Frage, die unter anderem den Glanzgrad der Oberfläche beeinflussen.

Die Ansaugöffnung des rohrförmigen Grundkörpers verläuft nicht rechtwinklig zur Längsachse, sondern ist schräg zu ihr ausgeführt. Dadurch ergibt sich eine schräg zulaufende Form des Spiegelsaugers, wodurch dieser leichter beispielsweise zwischen Wange und Zähne einführbar ist. Der Spiegel befindet sich nicht vor der Ansaugöffnung, sondern in Strömungsrichtung der angesaugten Luft im Wesentlichen hinter der Ansaugöffnung, also innerhalb des Grundkörpers. Hierdurch wird erreicht, dass der Spiegelsauger nicht durch einen vorgelagerten Spiegel verlängert wird, was die Ansaugleistung vermindern würde.

Obwohl ein Verkleben der Grundkörperteile miteinander grundsätzlich möglich ist, hat es sich als besonders vorteilhaft erwiesen, die beiden Grundkörperteile miteinander zu verschweißen und keinen Klebstoff zu verwenden. Die grundsätzlich mit Klebstoff verbundenen Nachteile können dadurch vermieden werden.

Das erfindungsgemäße Verfahren zur Herstellung des Spiegelsaugers weist die folgenden Verfahrensschritte auf
- Fertigen des ersten Grundkörperteils mit einer Spiegelaufnahme für den Spiegel, die die Öffnung mit einer im Wesentlichen umlaufenden oberen Halteschulter aufweist,
- Einsetzen des Spiegels in die Spiegelaufnahme derart, dass die obere Halteschulter an eine Spiegelfläche des Spiegels anliegt,
- Fertigen eines zweiten Grundkörperteils und Verbinden des zweiten Grundkörperteils mit dem ersten Grundkörperteil im Bereich der Spiegelrückseite, so dass die beiden Grundkörperteile einen einstückigen Grundkörper ausbilden, so dass die beiden Grundkörperteile nach der Fertigung nur durch Zerstören wieder voneinander getrennt werden können.

Vorteilhafterweise kann das erste Grundkörperteil derart gefertigt und ausgeführt werden, dass der Spiegel bereits vor der Verbindung der beiden Grundkörperteile miteinander reib- oder formschlüssig im ersten Grundkörperteil gehalten ist. Dies vereinfacht die nachfolgenden Fertigungsschritte.

Das Verfahren ist somit schnell und einfach durchzuführen. Aufgrund der relativ einfachen Grundform des zweiten Grundkörperteils ist die Konstruktion des Werkzeugs zur Fertigung ebenfalls eher einfach und die Zuverlässigkeit des Werkzeugs bei der Fertigung hoch.

Die Erfindung wird anhand der nachfolgenden Figuren näher erläutert. Diese zeigen lediglich Ausführungsbeispiele, die Erfindung soll nicht auf diese beschränkt sein.

Es zeigen:
- Fig. 1:: einen erfindungsgemäßen Spiegelsauger von oben,
- Fig. 2:: den erfindungsgemäßen Spiegelsauger aus Fig. 1 von der Seite,
- Fig. 3:: den erfindungsgemäßen Spiegelsauger aus Fig. 1 im Längsschnitt,
- Fig. 4:: eine Vergrößerung des Bereichs A aus Fig. 3,
- Fig. 5.:: einen Spiegel in Seitenansicht,
- Fig. 6:: eine vereinfachte Darstellung des vorderen Bereichs des Spiegelsaugers von unten.

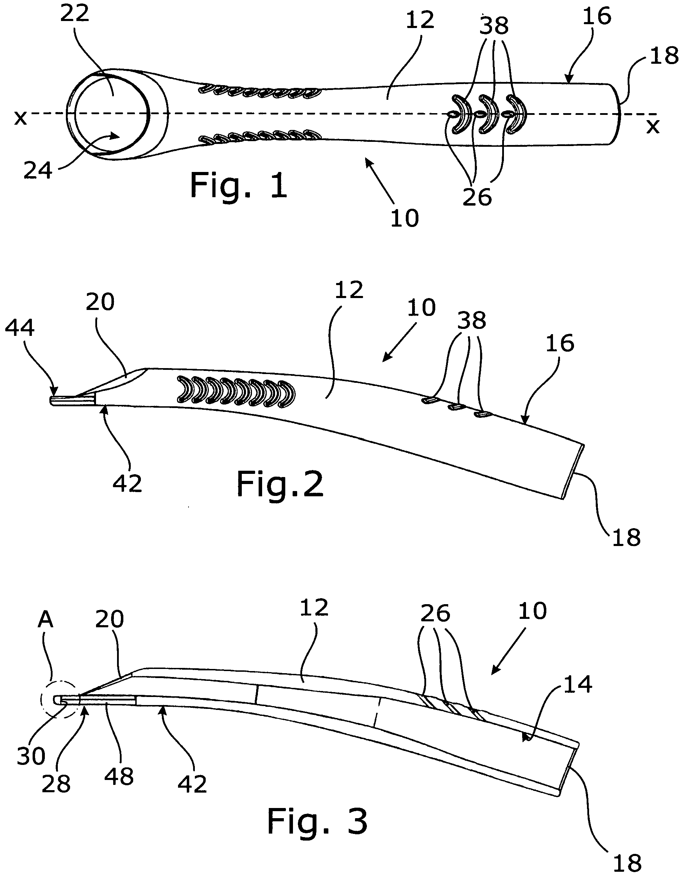

Wie sich insbesondere aus den Figuren 1 bis 3 ergibt, weist ein erfindungsgemäßer Spiegelsauger 10 einen hohlen rohrförmigen Grundkörper 12 mit einer Innenfläche 14 und einer Außenfläche 16 auf. Weiterhin weist der Grundkörper 12 eine Längsachse X-X auf (vergl. Fig. 1). Die insbesondere in den Figuren 2 und 3 erkennbare Bogenform des Spiegelsaugers 10 hat den Vorteil, dass dieser leichter an die zu behandelnde Stelle herangeführt werden kann.

Der Grundkörper 12 weist eine Anschlussöffnung 18 für einen nicht gezeigten Schlauch und eine Ansaugöffnung 20 zum Ansaugen von Partikeln und Flüssigkeiten auf. Durch die Ansaugöffnung 20 werden die abzusaugenden Flüssigkeiten oder Partikel eingesogen und durch die Anschlussöffnung 18 über den Schlauch abgeleitet.

Erfindungsgemäß ist innerhalb des Grundkörpers 12 ein Spiegel 22 im Bereich der Ansaugöffnung 20 angeordnet, der durch die Ansaugöffnung 20 einsehbar ist. Entsprechend ist eine sichtbare Spiegelfläche 24 der Ansaugöffnung 20 zugewandt. Der Spiegel 22 ist vollständig innerhalb des Grundkörpers 12, also in Strömungsrichtung der abzusaugende in Luft hinter der Ansaugöffnung 20 angeordnet ist. Die angesaugte Luft wird über die Spiegelfläche 24 geleitet, wodurch ein Beschlagen der Spiegelfläche 24 effektiv verhindert wird.

Der Spiegelsauger 10 weist Zusatzöffnungen 26 auf, durch die ebenfalls Luft angesaugten wird. Die Zusatzöffnungen 26 verhindern einen Unterdruck innerhalb des Grundkörpers 12, wenn die Ansaugöffnung 20 beispielsweise durch die Zunge oder Wange des Patienten verschlossen wird. Im gezeigten Ausführungsbeispiel sind drei Zusatzöffnungen 26 vorgesehen, denkbar ist aber auch nur eine einzige Zusatzöffnung 26 oder auch mehr als drei Zusatzöffnungen 26.

Auf der Außenfläche 16 des Grundkörpers 12 sind Profilelemente 38 erkennbar, die einen sicheren Griff des Spiegelsaugers 10 und ein Abrutschen der Finger des behandelnden Zahnarztes verhindern.

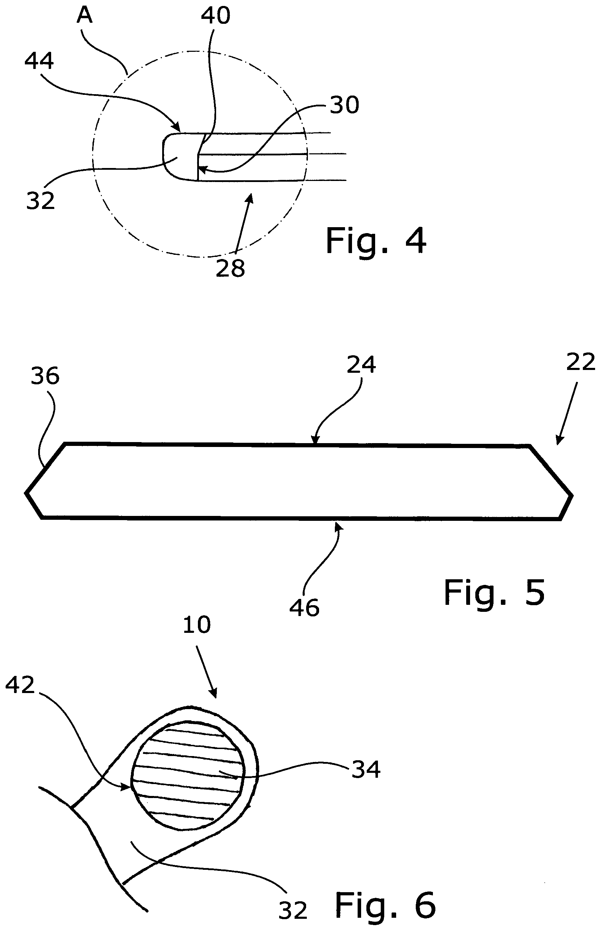

Die Figuren 3 und 6 zeigen dass der Grundkörper 12 aus einem ersten Grundkörperteil 32 und einem zweiten Grundkörperteil 34 gebildet ist. Das erste Grundkörperteil 32 weist eine Spiegelaufnahme 48 mit einer Öffnung 28 auf, in die der Spiegel 22 im zusammengesetzten Zustand eingesetzt ist. Eine Innenwandung 30 der Öffnung 28 umgibt den Spiegel 22 und liegt an einer Außenwandung 36 des Spiegels 22 zumindest bereichsweise an. Die Öffnung 28 verjüngt sich ausgehend von einer Grundkörperunterseite 42 in Richtung der Ansaugöffnung 20. Die Öffnung 28 weist auf ihrer der Grundkörperunterseite 42 zugewandten Seite einen Durchmesser auf der größer als der Durchmesser des Spiegels 22 ist. Dies ist Voraussetzung dafür, dass der Spiegel 22 in die Spiegelaufnahme 48 einführbar ist.

Der Spiegel 22 ist im Querschnitt zumindest abschnittsweise etwa trapezförmig ausgeführt (vgl. Fig. 5), so dass sein Durchmesser zumindest abschnittsweise ausgehend von der Spiegelfläche 24 in Richtung einer Grundkörperunterseite 42 zunimmt. Der Spiegel 22 weist eine der sichtbaren Spiegelfläche 24 abgewandte Spiegelrückseite 46 auf. In Fig. 5 ist eine Ausführungsform gezeigt, bei der der Spiegel 22 in vertikaler Richtung etwa im unteren Drittel einen maschinellen Durchmesser aufweist. Diese Form vereinfacht das Einsetzen oder Einklipsen in die Spiegelaufnahme 48. An den unteren Bereich der Außenwandung 36 liegt eine untere Halteschulter an.

Fig. 4 verdeutlicht in einer vergrößerten Darstellung des Bereichs A aus Fig. 4, dass eine obere Halteschulter 40 die gesamte Außenwandung 36 des Spiegels 22 umgibt, und einen Raum neben und unterhalb des Spiegels 22 abdichtet. Die Abdichtung wird über eine Vorspannung der oberen Halteschulter 40 verbessert. Dies bedeutet, dass der Spiegel 22 beim Einsetzen in das erste Grundkörperteil 32 gegen die obere Halteschulter 40 gedrückt wird und diese minimal komprimiert oder elastisch verformt wird.

Weiterhin hat diese Ausführungsvariante den Vorteil, dass der Spiegel 22 nahezu bündig in eine Bodenfläche 44 eingelassen ist.

Fig. 6 zeigt den vorderen Bereich des Spiegelsaugers 10 mit Sicht auf die Grundkörperunterseite 42. Erkennbar ist das zweite Grundkörperteil 34 (schraffiert), welcher die Öffnung 28 verschließt.

## Patentansprüche

1. Zahnmedizinischer Spiegelsauger (10) zum Absaugen von Flüssigkeiten und Partikeln aus einem Mundraum eines Patienten, mit einem rohrförmigen hohlen Grundkörper (12), der eine Innenfläche (14), eine Außenfläche (16), eine Längsachse (X-X), eine Anschlussöffnung (18) für einen Schlauch und eine Ansaugöffnung (20) aufweist, wobei
- die Innenfläche (14) einen durch die Ansaugöffnung (20) einsehbaren Spiegel (22) mit einer Spiegelfläche (24) und einer Spiegelrückseite (46) aufweist,
- der Grundkörper (12) aus einem ersten Grundkörperteil (32) und einem zweiten Grundkörperteil (34) gebildet ist,
- die beiden Grundkörperteile (32, 34) derart spaltfrei miteinander verbunden sind, dass sie gemeinsam den Grundkörper (12) einstückig ausbilden, so dass die beiden Grundkörperteile (32, 34) nur durch Zerstören voneinander getrennt werden können.
**dadurch gekennzeichnet, dass**
- der Spiegel (22) in einer Spiegelaufnahme (48) des ersten Grundkörperteils (32) in einer Öffnung (28) gehalten ist, deren Innenwandung (30) eine obere Halteschulter (40) ausbildet, die an einer Außenwandung (36) des Spiegels (24) anliegt, wobei die obere Halteschulter (40) durch das erste Grundkörperteil (32) ausgebildet ist und den gesamten Außenumfang des Spiegels (22) umgibt,
- das erste Grundkörperteil (32) nahezu den gesamten Grundkörper (12) des Spiegelsaugers (10) ausbildet und das zweite Grundkörperteil (34) im Bereich der Rückseite des Spiegels (22), also an der Unterseites des Spiegelsaugers (10) angeordnet ist und die Öffnung (28) verschließt,
- sich die Öffnung (28) des ersten Grundkörperteils (32) ausgehend von einer Grundkörperunterseite (42) in Richtung der Ansaugöffnung (20) verjüngt und auf ihrer der Grundkörperunterseite (42) zugewandten Seite einen Durchmesser aufweist, der größer als der Durchmesser des Spiegels (22) ist.

2. Zahnmedizinischer Spiegelsauger (10) nach Anspruch 1, **dadurch gekennzeichnet, dass** das zweite Grundkörperteil (34) die Spiegelrückseite (46) kontaktiert.

3. Zahnmedizinischer Spiegelsauger (10) nach Anspruch 1 oder Anspruch 2, **dadurch gekennzeichnet, dass** der Spiegel (20) kreisförmig ausgebildet ist.

4. Zahnmedizinischer Spiegelsauger (10) nach einem der Ansprüche 1 bis 3, **dadurch gekennzeichnet, dass** die Innenwandung (30) der Öffnung (28) eine untere Halteschulter ausbildet, die an einer Außenwandung (36) des Spiegels (22) anliegt.

5. Zahnmedizinischer Spiegelsauger (10) nach Anspruch 4, **dadurch gekennzeichnet, dass** das zweite Grundkörperteil (32) die untere Halteschulter ausbildet.

6. Zahnmedizinischer Spiegelsauger (10) nach einem der Ansprüche 1 bis 5, **dadurch gekennzeichnet, dass** der Spiegel (22) unverklebt in dem ersten Grundkörperteil (32) gehalten ist.

7. Zahnmedizinischer Spiegelsauger (10) nach einem der Ansprüche 1 bis 6, **dadurch gekennzeichnet, dass** die Grundkörperteile (32, 34) aus Kunststoff gefertigt sind.

8. Zahnmedizinischer Spiegelsauger (10) nach Anspruch 7, **dadurch gekennzeichnet, dass** die beiden Grundkörperteile (32, 34) miteinander verschweißt sind.

9. Zahnmedizinischer Spiegelsauger (10) nach Anspruch 7 oder Anspruch 8, **dadurch gekennzeichnet, dass** das zweite Grundkörperteil (34) an das erste Grundkörperteil (32) angespritzt ist.

10. Verfahren zur Herstellung eines zahnmedizinischen Spiegelsaugers (10) zum Absaugen von Flüssigkeiten und Partikeln aus einem Mundraum eines Patienten, mit einem hohlen Grundkörper (12), der eine Außenfläche (16), eine Innenfläche (14), eine Längsachse (X-X) und eine Ansaugöffnung (20) aufweist, wobei die Innenfläche (14) einen durch die Ansaugöffnung (20) einsehbaren Spiegel (22) aufweist,
**gekennzeichnet durch die** Verfahrensschritte
- Fertigen eines ersten Grundkörperteils (32) mit einer Spiegelaufnahme für den Spiegel (22), die eine Öffnung (28) mit einer im Wesentlichen umlaufenden oberen Halteschulter (40) aufweist,
- Einsetzen des Spiegels (22) in die Spiegelaufnahme derart, dass die obere Halteschulter (40) an eine Außenwandung (36) des Spiegels (22) anliegt,
- Fertigen eines zweiten Grundkörperteils (34) und Verbinden des zweiten Grundkörperteils (34) mit dem ersten Grundkörperteil (32) im Bereich der Spiegelrückseite (46), so dass das zweite Grundkörperteil (34) im Bereich der Rückseite des Spiegels (22), also an der Unterseite des Spiegelsaugers (10) angeordnet ist und die Öffnung (28) verschließt, so dass die beiden Grundkörperteile (32, 34) einen einstückigen Grundkörper (12) ausbilden, so dass die beiden Grundkörperteile (32, 34) nach der Fertigung nur durch Zerstören wieder voneinander getrennt werden können.

11. Verfahren nach Anspruch 10, **dadurch gekennzeichnet, dass** beide Grundkörperteile (32, 34) aus Kunststoff gefertigt werden und das zweite Grundkörperteil (34) an das erste Grundkörperteil (32) angespritzt wird.

## Claims

1. A dental mirror suction device (10) for suctioning liquids and particles from an oral cavity of a patient, with a tubular hollow basic body (12) having an inner surface (14), an outer surface (16), a longitudinal axis (X-X), a connection port (18) for a hose, and a suction port (20), wherein
- the inner surface (14) has a mirror (22) viewable through the suction port (20), with a mirror surface (24) and a back (46) of the mirror,
- the basic body (12) is formed from a first basic body part (32) and a second basic body part (34),
- the two basic body parts (32, 34) are connected to each other in a gap-free manner in such a way that, together, they integrally form the basic body (12), so that the two basic body parts (32, 34) can only be separated from each other destructively,
**characterized in that**
- the mirror (22) is retained in a mirror-accommodating portion (48) of the first basic body part (32) in an opening (28) whose inner wall (30) forms an upper retaining shoulder (40) abutting against an outer wall (36) of the mirror (24), wherein the upper retaining shoulder (40) is formed by the first basic body part (32) and surrounds the entire outer circumference of the mirror (22),
- the first basic body part (32) forms almost the entire basic body (12) of the mirror suction device (10), and the second body part (34) is disposed in the area of the back of the mirror (22), i.e. at the underside of the mirror suction device (10), and seals the opening (28),
- the opening (28) of the first basic body part (32) tapers from an underside (42) of the basic body in the direction towards the suction port (20) and has a diameter, on its side facing towards the underside (42) of the basic body, which is greater than the diameter of the mirror (22).

2. The dental mirror suction device (10) according to claim 1, **characterized in that** the second basic body part (34) contacts the back (46) of the mirror.

3. The dental mirror suction device (10) according to claim 1 or claim 2, **characterized in that** the mirror (20) is formed in a circular manner.

4. The dental mirror suction device (10) according to any one of the claims 1 to 3, **characterized in that** the inner wall (30) of the opening (28) forms a lower retaining shoulder abutting against the outer wall (36) of the mirror (22).

5. The dental mirror suction device (10) according to claim 4, **characterized in that** the second basic body part (32) forms the lower retaining shoulder.

6. The dental mirror suction device (10) according to any one of the claims 1 to 5, **characterized in that** the mirror (22) is retained without adhesive in the first basic body part (32).

7. The dental mirror suction device (10) according to any one of the claims 1 to 6, **characterized in that** the basic body parts (32, 34) are made from plastic.

8. The dental mirror suction device (10) according to claim 7, **characterized in that** the two basic body parts (32, 34) are welded to each other.

9. The dental mirror suction device (10) according to claim 7 or claim 8, **characterized in that** the second basic body part (34) is injection-molded to the first basic body part (32).

10. A method for producing a dental mirror suction device (10) for suctioning liquids and particles from an oral cavity of a patient, with a hollow basic body (12) having an outer surface (16), an inner surface (14), a longitudinal axis (X-X) and a suction port (20), wherein the inner surface (14) has a mirror (22) viewable through the suction port (20),
**characterized by the process steps**
- manufacturing a first basic body part (32) with a mirror-accommodating portion for the mirror (22), which has an opening (28) with a substantially peripheral upper retaining shoulder (40),
- inserting the mirror (22) into the mirror-accommodating portion in such a way that the upper retaining shoulder (40) abuts against an outer wall (36) of the mirror (22),
- manufacturing a second basic body part (34) and connecting the second basic body part (34) to the first basic body part (32) in the area of the back (46) of the mirror, so that the second basic body part (34) is disposed in the area of the back of the mirror (22), i.e. at the underside of the mirror suction device (10), and seals the opening (28), so that the two basic body part (32, 34) form an integral basic body (12), so that subsequent to manufacture, the two basic body parts (32, 34) can only be separated from each other again destructively.

11. The method according to claim 10, **characterized in that** both basic body parts (32, 34) are made from plastic, and the second basic body part (34) is injection-molded to the first basic body part (32).

## Revendications

1. Pompe à salive à miroir (10) de médecine dentaire destinée à aspirer des liquides et des particules d'une cavité buccale d'un patient, comprenant un corps de base (12) creux tubulaire qui présente une surface intérieure (14), une surface extérieure (16), un axe longitudinal (X-X), une ouverture de raccordement (18) pour un tuyau flexible ainsi qu'une ouverture d'aspiration (20), dans laquelle
- ladite surface intérieure (14) présente un miroir (22) visible à travers l'ouverture d'aspiration (20) et ayant une surface de miroir (24) ainsi qu'une face arrière de miroir (46),
- le corps de base (12) est constitué par une première partie de corps de base (32) et par une deuxième partie de corps de base (34),
- les deux parties de corps de base (32, 34) sont reliées l'une à l'autre sans jeu de telle manière qu'elles forment ensemble, d'un seul tenant, le corps de base (12) de sorte que les deux parties de corps de base (32, 34) ne puissent être séparées l'une de l'autre qu'en les détruisant,
**caractérisée par le fait que**
- le miroir (22) est retenu dans un logement (48) de la première partie de corps de base (32), dans une ouverture (28) dont la paroi intérieure (30) forme un épaulement supérieur de retenue (40) qui s'applique contre une paroi extérieure (36) du miroir (24), ledit épaulement supérieur de retenue (40) étant formé par la première partie de corps de base (32) et entourant toute la périphérie extérieure du miroir (22),
- la première partie de corps de base (32) forme presque tout le corps de base (12) de la pompe à salive à miroir (10), et la deuxième partie de corps de base (34) est agencée au niveau de la face arrière du miroir (22), donc sur la face inférieure de la pompe à salive à miroir (10), et ferme l'ouverture (28),
- l'ouverture (28) de la première partie de corps de base (32) se rétrécit en direction de l'ouverture d'aspiration (20), à partir d'une face inférieure (42) du corps de base, et présente sur son côté montrant vers la face inférieure (42) du corps de base un diamètre qui est supérieur au diamètre du miroir (22).

2. Pompe à salive à miroir (10) de médecine dentaire selon la revendication 1, **caractérisée par le fait que** la deuxième partie de corps de base (34) entre en contact avec la face arrière de miroir (46).

3. Pompe à salive à miroir (10) de médecine dentaire selon la revendication 1 ou la revendication 2, **caractérisée par le fait que** le miroir (22) est circulaire.

4. Pompe à salive à miroir (10) de médecine dentaire selon l'une quelconque des revendications 1 à 3, **caractérisée par le fait que** la paroi intérieure (30) de l'ouverture (28) forme un épaulement inférieur de retenue qui s'applique contre une paroi extérieure (36) du miroir (22).

5. Pompe à salive à miroir (10) de médecine dentaire selon la revendication 4, **caractérisée par le fait que** la deuxième partie de corps de base (34) forme l'épaulement inférieur de retenue.

6. Pompe à salive à miroir (10) de médecine dentaire selon l'une quelconque des revendications 1 à 5, **caractérisée par le fait que** le miroir (22) est maintenu sans être collé dans la première partie de corps de base (32).

7. Pompe à salive à miroir (10) de médecine dentaire selon l'une quelconque des revendications 1 à 6, **caractérisée par le fait que** les parties de corps de base (32, 34) sont réalisées en matière plastique.

8. Pompe à salive à miroir (10) de médecine dentaire selon la revendication 7, **caractérisée par le fait que** les deux parties de corps de base (32, 34) sont soudées l'une à l'autre.

9. Pompe à salive à miroir (10) de médecine dentaire selon la revendication 7 ou la revendication 8, **caractérisée par le fait que** la deuxième partie de corps de base (34) est moulée par injection sur la première partie de corps de base (32).

10. Procédé de fabrication d'une pompe à salive à miroir (10) de médecine dentaire destinée à aspirer des liquides et des particules d'une cavité buccale d'un patient, comprenant un corps de base (12) creux qui présente une surface extérieure (16), une surface intérieure (14), un axe longitudinal (X-X) et une ouverture d'aspiration (20), dans lequel la surface intérieure (14) présente un miroir (22) visible à travers ladite ouverture d'aspiration (20),
**caractérisé par** les étapes de procédé consistant à
- fabriquer une première partie de corps de base (32) ayant un logement de miroir pour le miroir (22), qui présente une ouverture (28) avec un épaulement supérieur de retenue (40) pour l'essentiel circonférentiel,
- insérer le miroir (22) dans le logement de miroir de telle sorte que ledit épaulement supérieur de retenue (40) s'applique contre une paroi extérieure (36) du miroir (22),
- fabriquer une deuxième partie de corps de base (34) et relier la deuxième partie de corps de base (34) à la première partie de corps de base (32) au niveau de la face arrière de miroir (46) de sorte que la deuxième partie de corps de base (34) est agencée au niveau de la face arrière du miroir (22), donc sur la face inférieure de la pompe à salive à miroir (10), et ferme l'ouverture (28), de sorte que les deux parties de corps de base (32, 34) forment un corps de base (12) en une seule pièce de sorte que, après avoir été fabriquées, les deux parties de corps de base (32, 34) ne peuvent être à nouveau séparées l'une de l'autre qu'en les détruisant.

11. Procédé selon la revendication 10, **caractérisé par le fait que** les deux parties de corps de base (32, 34) sont réalisées en matière plastique et que la deuxième partie de corps de base (34) est moulée par injection sur la première partie de corps de base (32).
